# EUROPEAN PATENT APPLICATION

(11) **EP 0 887 406 A2**
(43) Date of publication of application: **30.12.1998**
(21) Application number: 98303163.4
(22) Date of filing: 23.04.1998
(51) Int. Cl.: C12N 15/12, C12N 5/10, C07K 14/71, C07K 16/28, A61K 38/18, A61K 48/00

(54) **A member of the FRZB family, franzzled**

(30) Priority: 22.05.1997 US 47408 P; 26.11.1997 US 978981
(71) Applicant: SMITHKLINE BEECHAM CORPORATION, Philadelphia Pennsylvania 19103 (US)
(72) Inventor: Lark, Michael, King of Prussia, Pennsylvania 19406 (US); James, Ian, King of Prussia, Pennsylvania 19406 (US); Kumar, Sanjay, King of Prussia, Pennsylvania 19406 (US)
(74) Representative: Connell, Anthony Christopher

(57) **Abstract**

FRAZZLED polypeptides and polynucleotides and methods for producing such polypeptides by recombinant techniques are disclosed. Also disclosed are methods for utilizing FRAZZLED polypeptides and polynucleotides in the design of protocols for the treatment of chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, among others, and diagnostic assays for such conditions.

## Description

This application claims the benefit of U.S. Provisional Application No. 60/047,408, filed May 22, 1997.

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to FRZB family, hereinafter referred to as FRAZZLED. The invention also relates to inhibiting or activating the action of such polynucleotides and polypeptides.

### BACKGROUND OF THE INVENTION

Recently, a number of studies have focused on the identification and characterization of proteins which control developmental patterning. These proteins are members of a large family (referred to as the frizzled family), exemplified by frizzled and smoothened [Moon, et al., Cell 88: 725-728 (1997)]. Smoothened is a 7 transmembrane protein which associates with the 12 transmembrane protein, patched, to regulate signaling of the soluble agonist, indian hedgehog (Stone, et al., Science 384(14): 129-134 (1996)]. Indian hedgehog and parathyroid hormone-related peptide appear to regulate the differentiation of chondrocytes in mammalian systems [Vortkamp, et al. Science 273(2): 613-622 (1996)]. The control of the chondrocyte phenotype could be critically important in the maintenance of cartilage homeostasis in diseases involving both bone and cartilage including osteoarthritis, osteoporosis and rheumatoid arthritis.

In addition to the plasma membrane-associated members of the frizzled family, a soluble frizzled-related protein subfamily has recently been described. Members of this family, referred to as either Frzb, Fritz, frezzled or sFRPs (soluble frizzled related proteins), appear to control signaling by binding frizzled agonists, extracellularly [Moon, et al., Cell 88: 725-728 (1997); Wang, et al., Cell 88: 757-766 (1997); Leyns, et al., Cell 88: 747-756 (1997)]. The first description of Frzb was from extracts of bovine articular cartilage [Hoang, et al., J. Biol. Chem. 271(42): 26131-26137 (1996)]. In that study, it was reported that Frzb was expressed in chondrocytes in both developing cartilage rudiments and at sites of long bone growth. These authors also described the human Frzb homologue and reported that it is 94% identical to the bovine sequence. More recently, several sFRPs have been identified in the mouse [Rattner, et al., PNAS 94: 2859-2863 (1997)]. One member of this subfamily, sFRP-3 is 92% identical to bovine and human Frzb. When sFRP-3 was expressed as a construct containing a hydrophobic transmembrane domain, it had the ability to bind the frizzled agonist, wingless, confirming that the soluble mammalian sFRP-3 has the ability to bind frizzled agonists.

The numerous studies described above suggest that members of the frizzled family play key roles in cartilage and bone morphogenesis. However, it is unclear what role, if any, these proteins play in the maintenance of adult bone and/or cartilage. Consistent with a potential role in mature tissues, Frzb was originally isolated from calf articular cartilage. Furthermore, it has been proposed that at sites of active bone and cartilage remodeling, exemplified by osteoarthritis and fracture callus healing [Hughes, et al., J. Bone Miner. Res. 10(4): 533-544 (1995)], there may be differentiation of hypertrophic chondrocytes into osteoblast-like bone forming cells. Aberrant control of this process may result in the subchondral bone sclerosis observed in osteoarthritis, which may lead to the development and progression of this disease. This invention describes a novel member of the human Frzb family.

This indicates that the FRZB family has an established, proven history as therapeutic targets. Clearly there is a need for identification and characterization of further members of FRZB family which can play a role in preventing, ameliorating or correcting dysfunctions or diseases, including, but not limited to, chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease.

### SUMMARY OF THE INVENTION

In one aspect, the invention relates to FRAZZLED polypeptides and recombinant materials and methods for their production. Another aspect of the invention relates to methods for using such FRAZZLED polypeptides and polynucleotides. Such uses include the treatment of chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, among others. In still another aspect, the invention relates to methods to identify agonists and antagonists using the materials provided by the invention, and treating conditions associated with FRAZZLED imbalance with the identified compounds. Yet another aspect of the invention relates to diagnostic assays for detecting diseases associated with inappropriate FRAZZLED activity or levels.

### DESCRIPTION OF THE INVENTION

### Definitions

The following definitions are provided to facilitate understanding of certain terms used frequently herein.

"FRAZZLED" refers, among others, generally to a polypeptide having the amino acid sequence set forth in SEQ ID NO:2 or an allelic variant thereof.

"FRAZZLED activity or FRAZZLED polypeptide activity" or "biological activity of the FRAZZLED or FRAZZLED polypeptide" refers to the metabolic or physiologic function of said FRAZZLED including similar activities or improved activities or these activities with decreased undesirable side-effects. Also included are antigenic and immunogenic activities of said FRAZZLED.

"FRAZZLED gene" refers to a polynucleotide having the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single-and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al., Nucleic Acids Research* (1984) 12(1):387), BLASTP, BLASTN, FASTA (Atschul, S.F. *et al., J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to FRAZZLED polypeptides (or FRAZZLED proteins) . The FRAZZLED polypeptides include the polypeptide of SEQ ID NOS:2 and 4; as well as polypeptides comprising the amino acid sequence of SEQ ID NO: 2; and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within FRAZZLED polypeptides are polypeptides having the amino acid sequence which have at least 80% identity to the polypeptide having the amino acid sequence of SEQ ID NO:2 over its entire length, and still more preferably at least 90% identity, and still more preferably at least 95% identity to SEQ ID NO:2. Furthermore, those with at least 97-99% are highly preferred. Preferably FRAZZLED polypeptide exhibit at least one biological activity of FRAZZLED.

The FRAZZLED polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the FRAZZLED polypeptides are also included in the invention. A fragment is a polypeptide having an amino acid sequence that entirely is the same as part, but not all, of the amino acid sequence of the aforementioned FRAZZLED polypeptides. As with FRAZZLED polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of FRAZZLED polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of FRAZZLED polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate FRAZZLED activity, including those with a similar activity or an improved activity, or with a decreased undesirable activity. Also included are those that are antigenic or immunogenic in an animal, especially in a human.

Preferably, all of these polypeptide fragments retain the biological activity of the FRAZZLED, including antigenic activity. Among the most preferred fragment is that having the amino acid sequence of SEQ ID NO: 4. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The FRAZZLED polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to FRAZZLED polynucleotides. FRAZZLED polynucleotides include isolated polynucleotides which encode the FRAZZLED polypeptides and fragments, and polynucleotides closely related thereto. More specifically, FRAZZLED polynucleotide of the invention include a polynucleotide comprising the nucleotide sequence contained in SEQ ID NO:1 encoding a FRAZZLED polypeptide of SEQ ID NO:2, and polynucleotides having the particular sequences of SEQ ID NOS:1 and 3. FRAZZLED polynucleotides further include a polynucleotide comprising a nucleotide sequence that has at least 80% identity over its entire length to a nucleotide sequence encoding the FRAZZLED polypeptide of SEQ ID NO:2, and a polynucleotide comprising a nucleotide sequence that is at least 80% identical to that of SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under FRAZZLED polynucleotides are a nucleotide sequence which has sufficient identity to a nucleotide sequence contained in SEQ ID NO:1 to hybridize under conditions useable for amplification or for use as a probe or marker. The invention also provides polynucleotides which are complementary to such FRAZZLED polynucleotides.

FRAZZLED of the invention is structurally related to other proteins of the FRZB family, as shown by the results of sequencing the cDNA encoding human FRAZZLED. The cDNA sequence of SEQ ID NO:1 contains an open reading frame (nucleotide number 105 to 1208) encoding a polypeptide of 368 amino acids of SEQ ID NO:2. The amino acid sequence of Table 1 (SEQ ID NO:2) has about 50.8% identity (using FASTA) in 319 amino acid residues with mouse sFRP-3 (A. Ratner et al., Proc. Natl. Acad. Sci. U.S.A. 94, 2859-2863, 1997)

The nucleotide sequence of Table 1 (SEQ ID NO:1) has about 81.4% identity (using FASTA) in 582 nucleotide residues with mouse sFRP-4 (A. Ratner et al., Proc. Natl. Acad. Sci. U.S.A. 94, 2859-2863, 1997). Thus, FRAZZLED polypeptides and polynucleotides of the present invention are expected to have, inter alia, similar biological functions/properties to their homologous polypeptides and polynucleotides, and their utility is obvious to anyone skilled in the art.

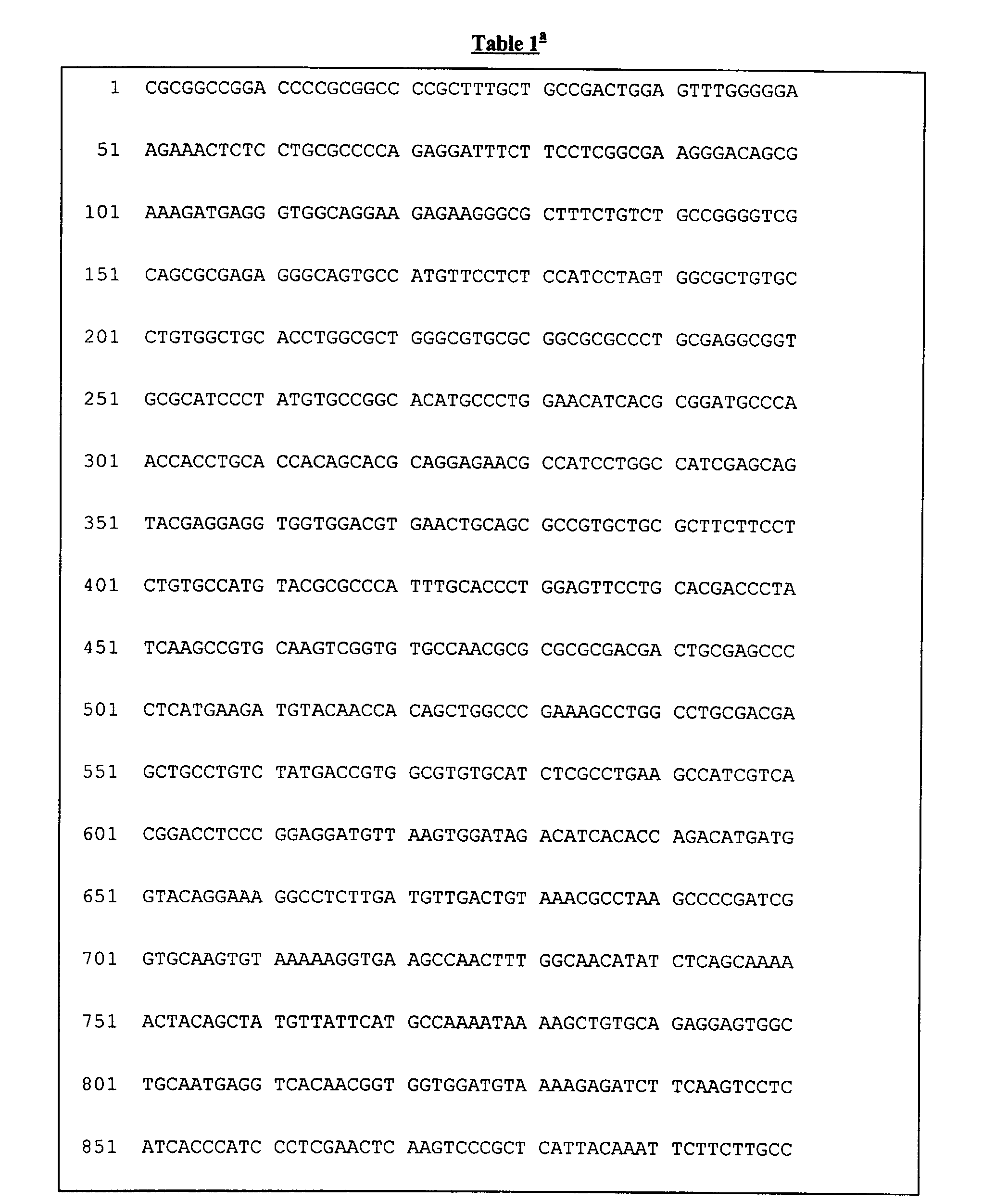

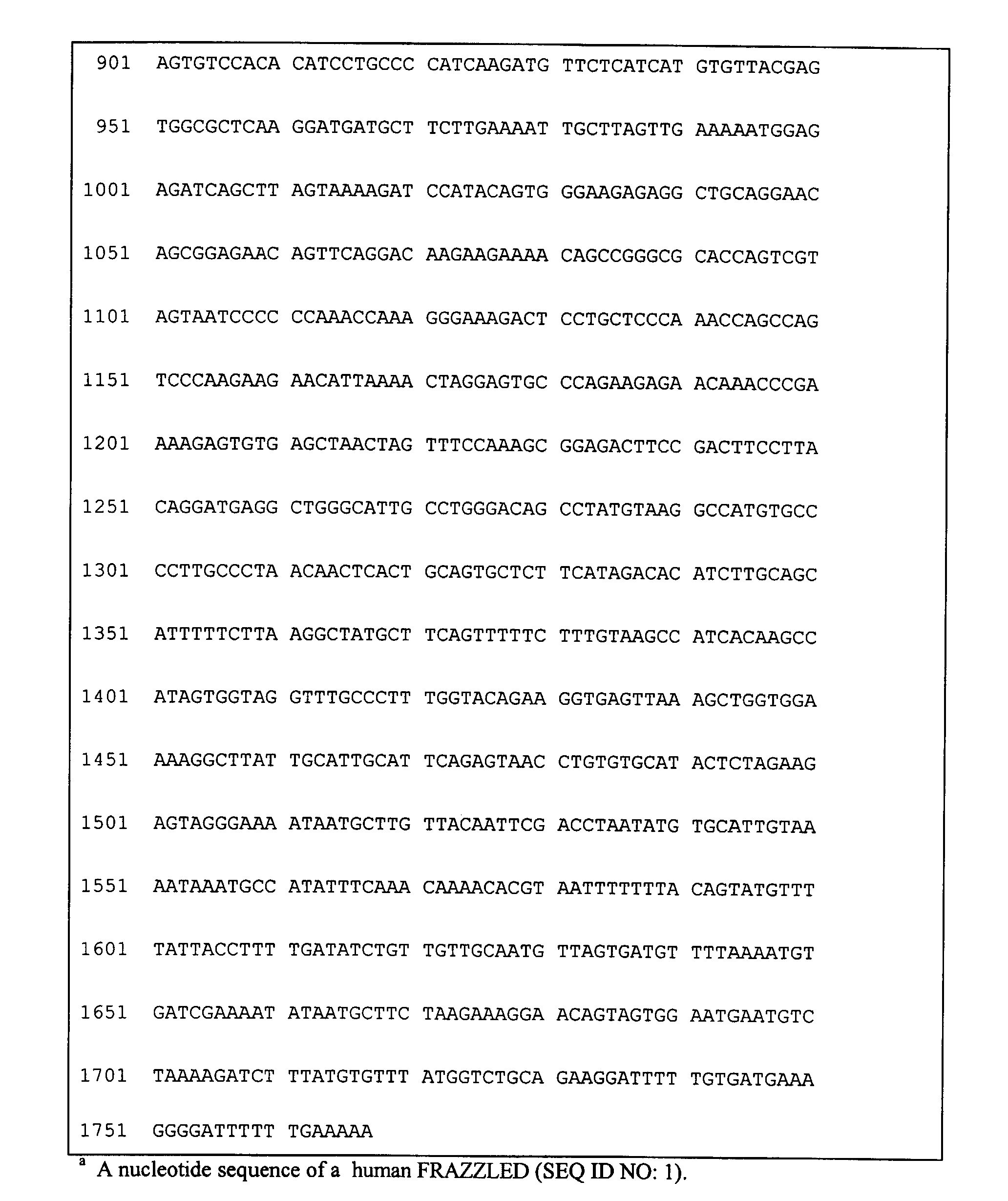

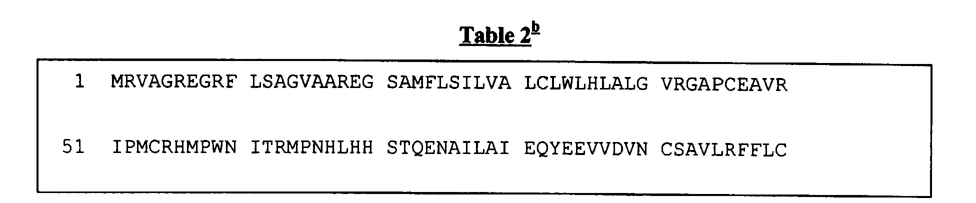

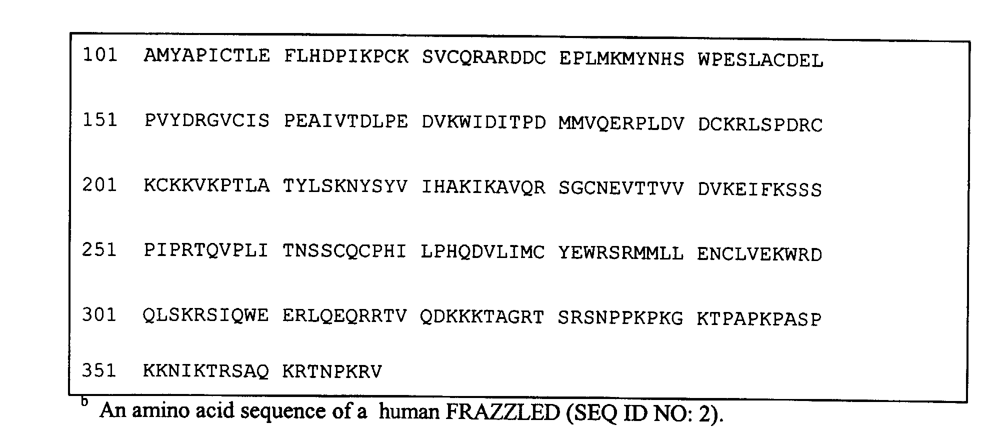

One polynucleotide of the present invention encoding FRAZZLED may be obtained using standard cloning and screening, from a cDNA library derived from mRNA in cells of human osteoblasts using the expressed sequence tag (EST) analysis (Adams, M.D., *et al. Science* (1991) 252:1651-1656; Adams, M.D. *et al., Nature,* (1992) *355:*632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding FRAZZLED polypeptide of SEQ ID NO:2 may be identical to the polypeptide encoding sequence contained in Table 1 (nucleotide number 105 to 1208 of SEQ ID NO:1), or it may be a sequence, which as a result of the redundancy (degeneracy) of the genetic code, also encodes the polypeptide of SEQ ID NO:2.

When the polynucleotides of the invention are used for the recombinant production of FRAZZLED polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding FRAZZLED variants comprising the amino acid sequence of FRAZZLED polypeptide of Table 2 (SEQ ID NO:2) in which several, 5-10, 1-5, 1-3, 1-2 or 1 amino acid residues are substituted, deleted or added, in any combination. Among the preferred polynucleotides of the present invention is contained in Table 3 (SEQ ID NO: 3) encoding the amino acid sequence of Table 4 (SEQ ID NO: 4).

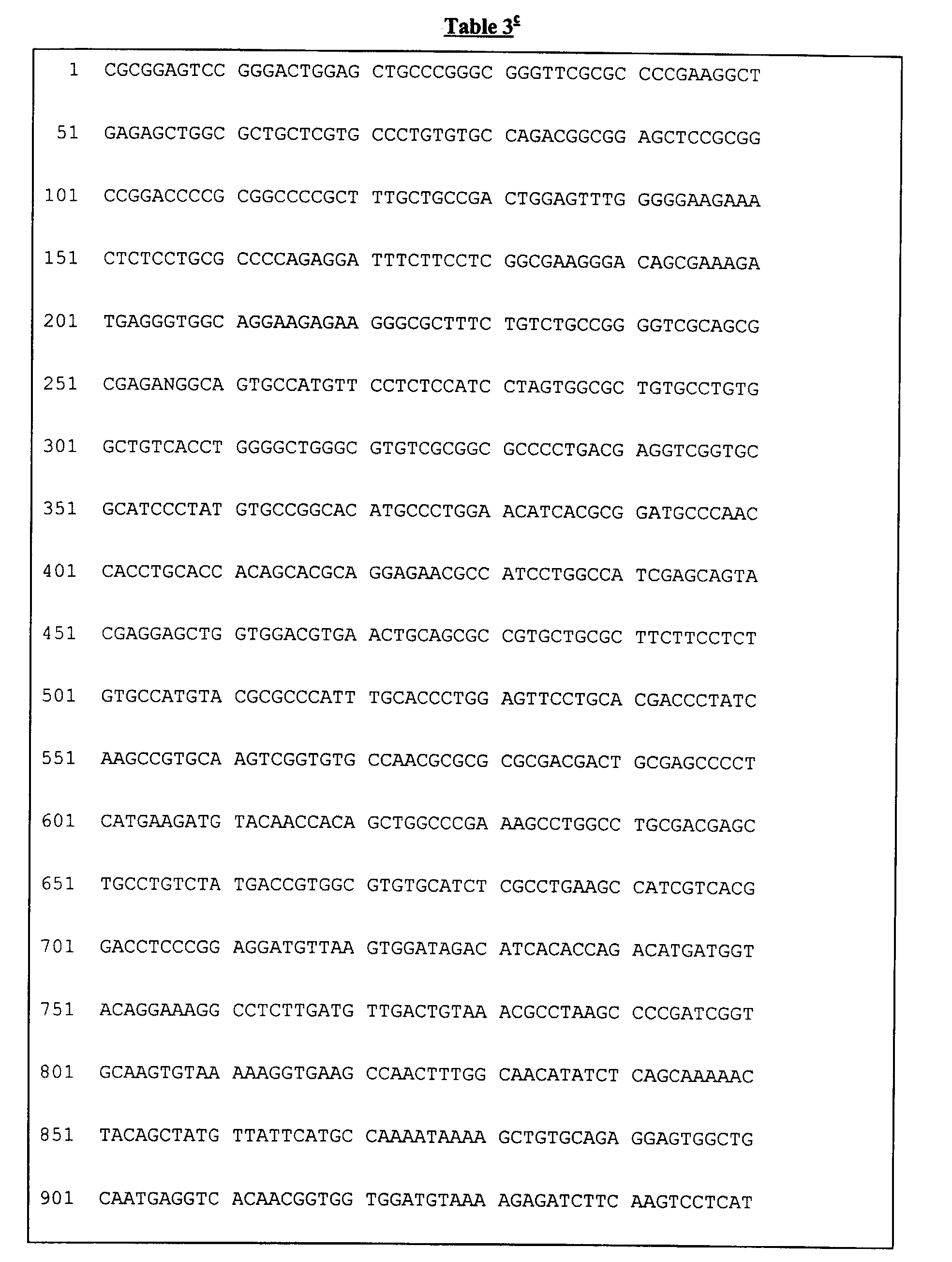

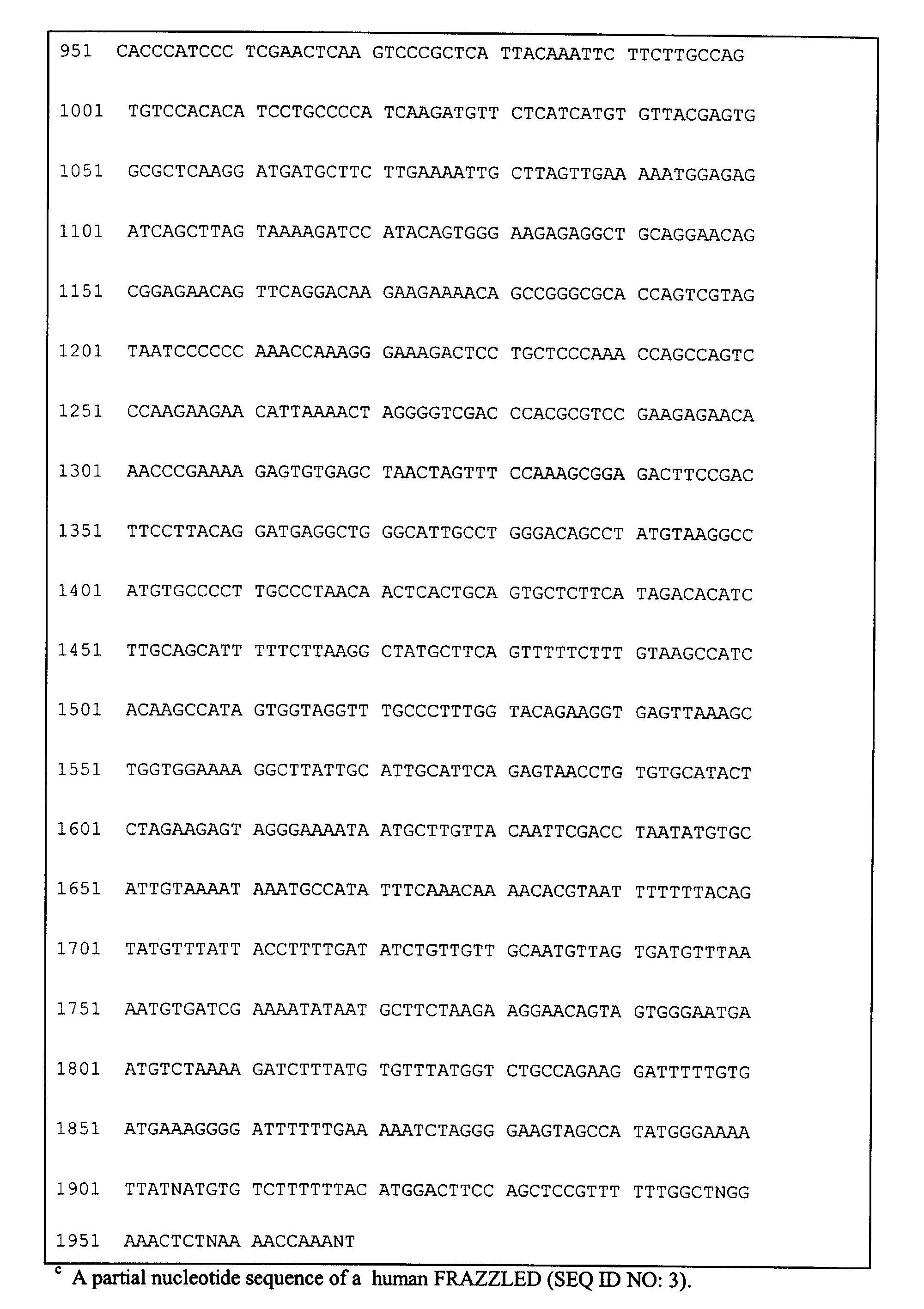

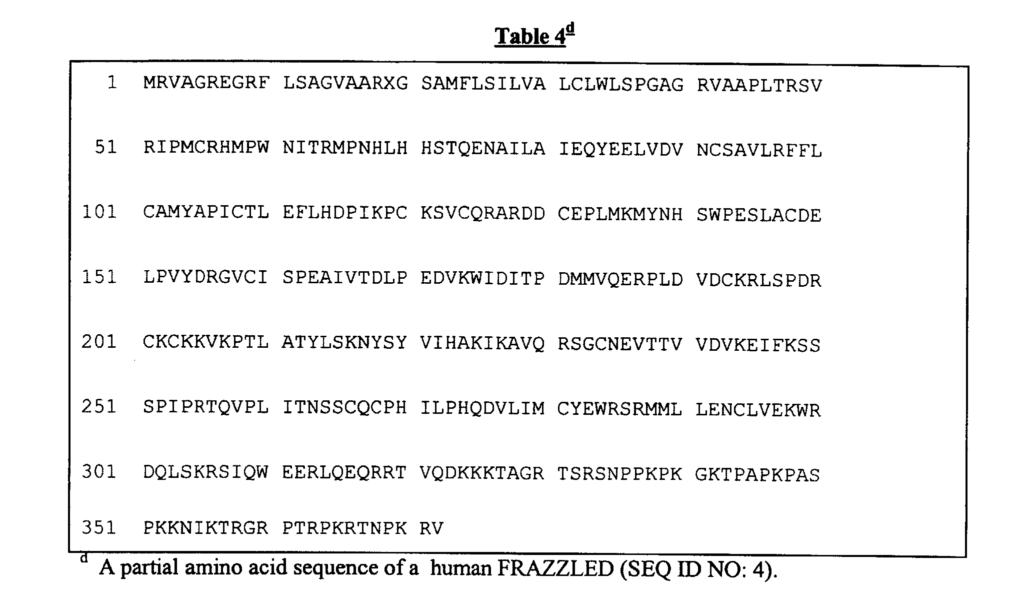

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 80%, and preferably at least 90%, and more preferably at least 95%, yet even more preferably 97-99% identity between the sequences.

Polynucleotides of the invention, which are identical or sufficiently identical to a nucleotide sequence contained in SEQ ID NO:1 or a fragment thereof (including that of SEQ ID NO:3), may be used as hybridization probes for cDNA and genomic DNA, to isolate full-length cDNAs and genomic clones encoding FRAZZLED polypeptide and to isolate cDNA and genomic clones of other genes (including genes encoding homologs and orthologs from species other than human) that have a high sequence similarity to the FRAZZLED gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding FRAZZLED polypeptide, including homologs and orthologs from species other than human, comprises the steps of screening an appropriate library under stingent hybridization conditions with a labeled probe having the SEQ ID NO: 1 or a fragment thereof (including that of SEQ ID NO: 3), and isolating full-length cDNA and genomic clones containing said polynucleotide sequence. Such hybridization techniques are well known to those of skill in the art. Thus in another aspect, FRAZZLED polynucleotides of the present invention further include a nucleotide sequence comprising a nucleotide sequence that hybridize under stringent condition to a nucleotide sequence having SEQ ID NO: 1 or a fragment thereof (including that of SEQ ID NO:3). Also included with FRAZZLED polypeptides are polypeptide comprising amino acid sequence encoded by nucleotide sequence obtained by the above hybridization condition. Stringent hybridization conditions are as defined above or, alternatively, conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL (supra).*

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the FRAZZLED polypeptide is to be expressed for use in screening assays, generally, it is preferred that the polypeptide be produced at the surface of the cell. In this event, the cells may be harvested prior to use in the screening assay. If FRAZZLED polypeptide is secreted into the medium, the medium can be recovered in order to recover and purify the polypeptide; if produced intracellularly, the cells must first be lysed before the polypeptide is recovered. FRAZZLED polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of FRAZZLED polynucleotides for use as diagnostic reagents. Detection of a mutated form of FRAZZLED gene associated with a dysfunction will provide a diagnostic tool that can add to or define a diagnosis of a disease or susceptibility to a disease which results from under-expression, over-expression or altered expression of FRAZZLED. Individuals carrying mutations in the FRAZZLED gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled FRAZZLED nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising FRAZZLED nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee *et al., Science,* Vol 274, pp 610-613 (1996)).

The diagnostic assays offer a process for diagnosing or determining a susceptibility to chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, through detection of mutation in the FRAZZLED gene by the methods described.

In addition, chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, can be diagnosed by methods comprising determining from a sample derived from a subject an abnormally decreased or increased level of FRAZZLED polypeptide or FRAZZLED mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an FRAZZLED polypeptide, in a sample derived from a host are well-known to those of skill in the art. Such assay methods include radioimmunoassays, competitive-binding assays, Western Blot analysis and ELISA assays.

Thus in another aspect, the present invention relates to a diagonostic kit for a disease or suspectability to a disease, particularly chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, which comprises:
(a) a FRAZZLED polynucleotide, preferably the nucleotide sequence of SEQ ID NO: 1, or a fragment thereof;
(b) a nucleotide sequence complementary to that of (a);
(c) a FRAZZLED polypeptide, preferably the polypeptide of SEQ ID NO: 2, or a fragment thereof; or
(d) an antibody to a FRAZZLED polypeptide, preferably to the polypeptide of SEQ ID NO: 2. It will be appreciated that in any such kit, (a), (b), (c) or (d) may comprise a substantial component.

### Chromosome Assays

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes). The differences in the cDNA or genomic sequence between affected and unaffected individuals can also be determined. If a mutation is observed in some or all of the affected individuals but not in any normal individuals, then the mutation is likely to be the causative agent of the disease.

The gene coding for FRAZZLED has been localized to the 15q21-23 locus.

### Antibodies

The polypeptides of the invention or their fragments or analogs thereof, or cells expressing them can also be used as immunogens to produce antibodies immunospecific for the FRAZZLED polypeptides. The term "immunospecific" means that the antibodies have substantiall greater affinity for the polypeptides of the invention than their affinity for other related polypeptides in the prior art.

Antibodies generated against the FRAZZLED polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495- 497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against FRAZZLED polypeptides may also be employed to treat chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, among others.

### Vaccines

Another aspect of the invention relates to a method for inducing an immunological response in a mammal which comprises inoculating the mammal with FRAZZLED polypeptide, or a fragment thereof, adequate to produce antibody and/or T cell immune response to protect said animal from chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, among others. Yet another aspect of the invention relates to a method of inducing immunological response in a mammal which comprises, delivering FRAZZLED polypeptide via a vector directing expression of FRAZZLED polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a FRAZZLED polypeptide wherein the composition comprises a FRAZZLED polypeptide or FRAZZLED gene. The vaccine formulation may further comprise a suitable carrier. Since FRAZZLED polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The FRAZZLED polypeptide of the present invention may be employed in a screening process for compounds which activate (agonists) or inhibit activation of (antagonists, or otherwise called inhibitors) the FRAZZLED polypeptide of the present invention. Thus, polypeptides of the invention may also be used to assess identify agonist or antagonists from, for example, cells, cell-free preparations, chemical libraries, and natural product mixtures. These agonists or antagonists may be natural or modified substrates, ligands, enzymes, receptors, etc., as the case may be, of the polypeptide of the present invention; or may be structural or functional mimetics of the polypeptide of the present invention. See Coligan *et al., Current Protocols in Immunology* 1(2):Chapter 5 (1991).

FRAZZLED polypeptides are responsible for many biological functions, including many pathologies. Accordingly, it is desirous to find compounds and drugs which stimulate FRAZZLED polypeptide on the one hand and which can inhibit the function of FRAZZLED polypeptide on the other hand. In general, agonists are employed for therapeutic and prophylactic purposes for such conditions as chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease.

Antagonists may be employed for a variety of therapeutic and prophylactic purposes for such conditions as chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease.

In general, such screening procedures may involve using appropriate cells which express the FRAZZLED polypeptide or respond to FRAZZLED polypeptide of the present invention. Such cells include cells from mammals, yeast, *Drosophila* or *E. coli.* Cells which express the FRAZZLED polypeptide (or cell membrane containing the expressed polypeptide) or respond to FRAZZLED polypeptide are then contacted with a test compound to observe binding, or stimulation or inhibition of a functional response. The ability of the cells which were contacted with the candidate compounds is compared with the same cells which were not contacted for FRAZZLED activity.

The FRAZZLED cDNA, protein and antibodies to the protein may also be used to configure assays for detecting the effect of added compounds on the production of FRAZZLED mRNA and protein in cells. For example, an ELISA may be constructed for measuring secreted or cell associated levels of FRAZZLED protein using monoclonal and polyclonal antibodies by standard methods known in the art, and this can be used to discover agents (i.e. antagonists or agonists) which may inhibit or enhance the production of FRAZZLED from suitably manipulated cells or tissues.

The FRAZZLED protein may be used to identify membrane bound or soluble ligand or receptors through standard ligand/receptor binding techniques known in the art. These include, but are not limited to, ligand binding and crosslinking assays in which the FRAZZLED is labeled with a radioactive isotope (e.g., 125I), chemically modified (e.g., biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. In addition to being used for purification and cloning of the receptor, these binding assays can be used to identify agonists and antagonists of FRAZZLED which compete with the binding of FRAZZLED to its receptors or ligands.

The above binding assays can be used to identify cells which respond biologically to FRAZZLED. Cells which respond to FRAZZLED may show changes in intracellular signal transduction pathways and in gene expression. These changes can be used in screens for agonists or antagonists which mimic or inhibit the action of FRAZZLED, respectively.

The assays may simply test binding of a candidate compound wherein adherence to the cells bearing the FRAZZLED polypeptide is detected by means of a label directly or indirectly associated with the candidate compound or in an assay involving competition with a labeled competitor. Further, these assays may test whether the candidate compound results in a signal generated by activation of the FRAZZLED polypeptide, using detection systems appropriate to the cells bearing the FRAZZLED polypeptide. Inhibitors of activation are generally assayed in the presence of a known agonist and the effect on activation by the agonist by the presence of the candidate compound is observed.

Further, the assays may simply comprise the steps of mixing a candidate compound with a solution containing a FRAZZLED polypeptide to form a mixture, measuring FRAZZLED activity in the mixture, and comparing the FRAZZLED activity of the mixture to a standard.

The FRAZZLED cDNA, protein and antibodies to the protein may also be used to configure assays for detecting the effect of added compounds on the production of FRAZZLED mRNA and protein in cells. For example, an ELISA may be constructed for measuring secreted or cell associated levels of FRAZZLED protein using monoclonal and polyclonal antibodies by standard methods known in the art, and this can be used to discover agents which may inhibit or enhance the production of FRAZZLED (also called antagonist or agonist, respectively) from suitably manipulated cells or tissues.

The FRAZZLED protein may be used to identify membrane bound or soluble receptors, if any, through standard receptor binding techniques known in the art. These include, but are not limited to, ligand binding and crosslinking assays in which the FRAZZLED is labeled with a radioactive isotope (e.g., 125I), chemically modified (e.g., biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. In addition to being used for purification and cloning of the receptor, these binding assays can be used to identify agonists and antagonists of FRAZZLED which compete with the binding of FRAZZLED to its receptors, if any. Standard methods for conducting screening assays are well understood in the art.

Examples of potential FRAZZLED polypeptide antagonists include antibodies or, in some cases, oligonucleotides or proteins which are closely related to the ligands, substrates, enzymes, receptors, etc., as the case may be, of the FRAZZLED polypeptide, e.g., a fragment of the ligands, substrates, enzymes, receptors, etc.; or small molecules which bind to the polypeptide of the present invention but do not elicit a response, so that the activity of the polypeptide is prevented.

Thus in another aspect, the present invention relates to a screening kit for identifying agonists, antagonists, ligands, receptors, substrates, enzymes, etc. for FRAZZLED polypeptides; or compounds which decrease or enhance the production of FRAZZLED polypeptides, which comprises:
(a) a FRAZZLED polypeptide, preferably that of SEQ ID NO:2;
(b) a recombinant cell expressing a FRAZZLED polypeptide, preferably that of SEQ ID NO:2;
(c) a cell membrane expressing a FRAZZLED polypeptide; preferably that of SEQ ID NO: 2; or
(d) antibody to a FRAZZLED polypeptide, preferably that of SEQ ID NO: 2.
It will be appreciated that in any such kit, (a), (b), (c) or (d) may comprise a substantial component.

### Prophylactic and Therapeutic Methods

This invention provides methods of treating abnormal conditions such as, chronic and acute inflammation, arthritis, osteoarthritis, septicemia, autoimmune diseases (e.g., inflammatory bowel disease, psoriasis), transplant rejection, graft vs. host disease, infection, stroke, ischemia, acute respiratory disease syndrome, renal disorders, restenosis, brain injury, AIDS, metabolic and other bone diseases (e.g., osteoporosis), cancer (e.g., lymphoproliferative disorders), atherosclerosis, and Alzheimers disease, related to both an excess of and insufficient amounts of FRAZZLED polypeptide activity.

If the activity of FRAZZLED polypeptide is in excess, several approaches are available. One approach comprises administering to a subject an inhibitor compound (antagonist) as hereinabove described along with a pharmaceutically acceptable carrier in an amount effective to inhibit the function of the FRAZZLED polypeptide, such as, for example, by blocking the binding of ligands, substrates, enzymes, receptors, etc., or by inhibiting a second signal, and thereby alleviating the abnormal condition. In another approach, soluble forms of FRAZZLED polypeptides still capable of binding the ligand, substrate, enzymes, receptors, etc. in competition with endogenous FRAZZLED polypeptide may be administered. Typical embodiments of such competitors comprise fragments of the FRAZZLED polypeptide.

In another approach, soluble forms of FRAZZLED polypeptides still capable of binding the ligand in competition with endogenous FRAZZLED polypeptide may be administered. Typical embodiments of such competitors comprise fragments of the FRAZZLED polypeptide.

In still another approach, expression of the gene encoding endogenous FRAZZLED polypeptide can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo.*

For treating abnormal conditions related to an under-expression of FRAZZLED and its activity, several approaches are also available. One approach comprises administering to a subject a therapeutically effective amount of a compound which activates FRAZZLED polypeptide, i.e., an agonist as described above, in combination with a pharmaceutically acceptable carrier, to thereby alleviate the abnormal condition. Alternatively, gene therapy may be employed to effect the endogenous production of FRAZZLED by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996). Another approach is to administer a therapeutic amount of FRAZZLED polypeptides in combination with a suitable pharmaceutical carrier.

### Formulation and Administration

Peptides, such as the soluble form of FRAZZLED polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

### Example 1

A partial clone encoding FRAZZLED (EST # 2105409) was identified through a search of a commercial EST database using the amino acid sequence of a previously identified member of the Frizzled family, Frzb. This clone was then fully sequenced and the full length sequence of this clone shared 73.8% identity with human Frzb (Hoang, et al., J. Biol. Chem. 271(42): 26131-26137 (1996)]. The clone encoding FRAZZLED was found in an osteoblast cell library. This gene is also expressed in chondrosarcoma, osteosarcoma, osteoclastoma, synovial fibroblasts, hodgkin's lymphoma, ovary, uterus, fetal lung, adipose and pancreatic tumor. Two ESTs corresponding to this gene from Soares NhHMPu S1 cDNA libraries are found in the public EST database.

### Example 2- Tissue distribution of FRAZZLED gene expression

Northern blot analysis was carried out to examine the expression of FRAZZLED mRNA expression in human tissues. A human multiple cell and multiple tissue northern blot (Clonetech Laboratories, Inc.) were hybridized with the entire nucleotide sequence of FRAZZLED cDNA labeled with ³²P using the rediprime DNA labeling system™ (Amersham Life Sciences), according to manufacturer's instructiuons. Hybridization and washes were carried out according to manufacturer's instructiuons and the blot was exposed to film at -70°C for 72 hours. FRAZZLED was expressed at high levels in ovary, testes and spleen. It was also expressed moderately in prostate, small intestine, colon, skeletal muscle and heart and at much lower levels in thymus, placenta, lung, kidney and pancreas.

All publications, including but not limited to patents and patent applications, cited in this specification are herein incorporated by reference as if each individual publication were specifically and individually indicated to be incorporated by reference herein as though fully set forth.

## Claims

1. An isolated polynucleotide comprising a nucleotide sequence that has at least 80% identity over its entire length to a nucleotide sequence encoding the FRAZZLED polypeptide of SEQ ID NO:2; or a nucleotide sequence complementary to said isolated polynucleotide.

2. The polynucleotide of claim 1 wherein said polynucleotide comprises the nucleotide sequence contained in SEQ ID NO:1 encoding the FRAZZLED polypeptide of SEQ ID NO2.

3. The polynucleotide of claim 1 wherein said polynucleotide comprises a nucleotide sequence that is at least 80% identical to that of SEQ ID NO: 1 over its entire length.

4. The polynucleotide of claim 3 which is polynucleotide of SEQ ID NO: 1.

5. The polynucleotide of claim 1 which is DNA or RNA.

6. A DNA or RNA molecule comprising an expression system, wherein said expression system is capable of producing a FRAZZLED polypeptide comprising an amino acid sequence, which has at least 80% identity with the polypeptide of SEQ ID NO:2 when said expression system is present in a compatible host cell.

7. A host cell comprising the expression system of claim 6.

8. A process for producing a FRAZZLED polypeptide comprising culturing a host of claim 7 under conditions sufficient for the production of said polypeptide and recovering the polypeptide from the culture.

9. A process for producing a cell which produces a FRAZZLED polypeptide thereof comprising transforming or transfecting a host cell with the expression system of claim 6 such that the host cell, under appropriate culture conditions, produces a FRAZZLED polypeptide.

10. A FRAZZLED polypeptide comprising an amino acid sequence which is at least 80% identical to the amino acid sequence of SEQ ID NO:2 over its entire length.

11. The polypeptide of claim 10 which comprises the amino acid sequence of SEQ ID NO:2.

12. An antibody immunospecific for the FRAZZLED polypeptide of claim 10.

13. A method for the treatment of a subject in need of enhanced activity or expression of FRAZZLED polypeptide of claim 10 comprising:
(a) administering to the subject a therapeutically effective amount of an agonist to said polypeptide; and/or
(b) providing to the subject an isolated polynucleotide comprising a nucleotide sequence that has at least 80% identity to a nucleotide sequence encoding the FRAZZLED polypeptide of SEQ ID NO:2 over its entire length; or a nucleotide sequence complementary to said nucleotide sequence in a form so as to effect production of said polypeptide activity *in vivo.*

14. A method for the treatment of a subject having need to inhibit activity or expression of FRAZZLED polypeptide of claim 10 comprising:
(a) administering to the subject a therapeutically effective amount of an antagonist to said polypeptide; and/or
(b) administering to the subject a nucleic acid molecule that inhibits the expression of the nucleotide sequence encoding said polypeptide; and/or
(c) administering to the subject a therapeutically effective amount of a polypeptide that competes with said polypeptide for its ligand, substrate , or receptor.

15. A process for diagnosing a disease or a susceptibility to a disease in a subject related to expression or activity of FRAZZLED polypeptide of claim 10 in a subject comprising:
(a) determining the presence or absence of a mutation in the nucleotide sequence encoding said FRAZZLED polypeptide in the genome of said subject; and/or
(b) analyzing for the presence or amount of the FRAZZLED polypeptide expression in a sample derived from said subject.

16. A method for identifying compounds which inhibit (antagonize) or agonize the FRAZZLED polypeptide of claim 10 which comprises:
(a) contacting a candidate compound with cells which express the FRAZZLED polypeptide (or cell membrane expressing FRAZZLED polypeptide) or respond to FRAZZLED polypeptide; and
(b) observing the binding, or stimulation or inhibition of a functional response; or comparing the ability of the cells (or cell membrane) which were contacted with the candidate compounds with the same cells which were not contacted for FRAZZLED polypeptide activity.

17. An agonist identified by the method of claim 16.

18. An antagonist identified by the method of claim 16.

19. A recombinant host cell produced by a method of Claim 9 or a membrane thereof expressing a FRAZZLED polypeptide.


### Annex to the description

SEQUENCE LISTING

(1) GENERAL INFORMATION

(i) APPLICANT: SmithKline Beecham Corporation

(ii) TITLE OF THE INVENTION: A MEMBER OF THE FRZB FAMILY,
FRAZZLED

(iii) NUMBER OF SEQUENCES: 4

(iv) CORRESPONDENCE ADDRESS:
(A) ADDRESSEE: SmithKline Beecham, Corporate Intellectual
Property
(B) STREET: Two New Horizons Court
(C) CITY: Brentford
(D) STATE: Middlesex
(E) COUNTRY: United Kingdom
(F) ZIP: TW8 9EP

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER: TO BE ASSIGNED
(B) FILING DATE: 26-NOV-1997
(C) CLASSIFICATION: UNKNOWN

(vii) PRIOR APPLICATION DATA:
(A) APPLICATION NUMBER: 60/047,408
(B) FILING DATE: 22-MAY-1997

(viii) ATTORNEY/AGENT INFORMATION:
(A) NAME: CONNELL, Anthony Christopher
(B) REGISTRATION NUMBER: 5630
(C) REFERENCE/DOCKET NUMBER: GH70035

(ix) TELECOMMUNICATION INFORMATION:
(A) TELEPHONE: +44 1279 633 395
(B) TELEFAX: +44 181 075 6294
(C) TELEX:

(2) INFORMATION FOR SEQ ID NO:1:

(i) SEQUENCE CHARACTERISTICS:
(A) LENGTH: 1767 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:1:

CGCGGCCGGA CCCCGCGGCC CCGCTTTGCT GCCGACTGGA GTTTGGGGGA AGAAACTCTC 60
CTGCGCCCCA GAGGATTTCT TCCTCGGCGA AGGGACAGCG AAAGATGAGG GTGGCAGGAA 120
GAGAAGGGCG CTTTCTGTCT GCCGGGGTCG CAGCGCGAGA GGGCAGTGCC ATGTTCCTCT 180
CCATCCTAGT GGCGCTGTGC CTGTGGCTGC ACCTGGCGCT GGGCGTGCGC GGCGCGCCCT 240
GCGAGGCGGT GCGCATCCCT ATGTGCCGGC ACATGCCCTG GAACATCACG CGGATGCCCA 300
ACCACCTGCA CCACAGCACG CAGGAGAACG CCATCCTGGC CATCGAGCAG TACGAGGAGG 360
TGGTGGACGT GAACTGCAGC GCCGTGCTGC GCTTCTTCCT CTGTGCCATG TACGCGCCCA 420
TTTGCACCCT GGAGTTCCTG CACGACCCTA TCAAGCCGTG CAAGTCGGTG TGCCAACGCG 480
CGCGCGACGA CTGCGAGCCC CTCATGAAGA TGTACAACCA CAGCTGGCCC GAAAGCCTGG 540
CCTGCGACGA GCTGCCTGTC TATGACCGTG GCGTGTGCAT CTCGCCTGAA GCCATCGTCA 600
CGGACCTCCC GGAGGATGTT AAGTGGATAG ACATCACACC AGACATGATG GTACAGGAAA 660
GGCCTCTTGA TGTTGACTGT AAACGCCTAA GCCCCGATCG GTGCAAGTGT AAAAAGGTGA 720
AGCCAACTTT GGCAACATAT CTCAGCAAAA ACTACAGCTA TGTTATTCAT GCCAAAATAA 780
AAGCTGTGCA GAGGAGTGGC TGCAATGAGG TCACAACGGT GGTGGATGTA AAAGAGATCT 840
TCAAGTCCTC ATCACCCATC CCTCGAACTC AAGTCCCGCT CATTACAAAT TCTTCTTGCC 900
AGTGTCCACA CATCCTGCCC CATCAAGATG TTCTCATCAT GTGTTACGAG TGGCGCTCAA 960
GGATGATGCT TCTTGAAAAT TGCTTAGTTG AAAAATGGAG AGATCAGCTT AGTAAAAGAT 1020
CCATACAGTG GGAAGAGAGG CTGCAGGAAC AGCGGAGAAC AGTTCAGGAC AAGAAGAAAA 1080
CAGCCGGGCG CACCAGTCGT AGTAATCCCC CCAAACCAAA GGGAAAGACT CCTGCTCCCA 1140
AACCAGCCAG TCCCAAGAAG AACATTAAAA CTAGGAGTGC CCAGAAGAGA ACAAACCCGA 1200
AAAGAGTGTG AGCTAACTAG TTTCCAAAGC GGAGACTTCC GACTTCCTTA CAGGATGAGG 1260
CTGGGCATTG CCTGGGACAG CCTATGTAAG GCCATGTGCC CCTTGCCCTA ACAACTCACT 1320
GCAGTGCTCT TCATAGACAC ATCTTGCAGC ATTTTTCTTA AGGCTATGCT TCAGTTTTTC 1380
TTTGTAAGCC ATCACAAGCC ATAGTGGTAG GTTTGCCCTT TGGTACAGAA GGTGAGTTAA 1440
AGCTGGTGGA AAAGGCTTAT TGCATTGCAT TCAGAGTAAC CTGTGTGCAT ACTCTAGAAG 1500

AGTAGGGAAA ATAATGCTTG TTACAATTCG ACCTAATATG TGCATTGTAA AATAAATGCC 1560
ATATTTCAAA CAAAACACGT AATTTTTTTA CAGTATGTTT TATTACCTTT TGATATCTGT 1620
TGTTGCAATG TTAGTGATGT TTTAAAATGT GATCGAAAAT ATAATGCTTC TAAGAAAGGA 1680
ACAGTAGTGG AATGAATGTC TAAAAGATCT TTATGTGTTT ATGGTCTGCA GAAGGATTTT 1740
TGTGATGAAA GGGGATTTTT TGAAAAA 1767

(2) INFORMATION FOR SEQ ID NO:2:

(i) SEQUENCE CHARACTERISTICS:
(A) LENGTH: 368 amino acids
(B) TYPE: amino acid
(C) STRANDEDNESS: single
(D) TOPOLOGY: linear
(ii) MOLECULE TYPE: protein

(xi) SEQUENCE DESCRIPTION: SEQ ID NO:2:

Met Arg Val Ala Gly Arg Glu Gly Arg Phe Leu Ser Ala Gly Val Ala
1               5                   10                  15
Ala Arg Glu Gly Ser Ala Met Phe Leu Ser Ile Leu Val Ala Leu Cys
            20                  25                  30
Leu Trp Leu His Leu Ala Leu Gly Val Arg Gly Ala Pro Cys Glu Ala
        35                  40                  45
Val Arg Ile Pro Met Cys Arg His Met Pro Trp Asn Ile Thr Arg Met
    50                  55                  60
Pro Asn His Leu His His Ser Thr Gln Glu Asn Ala Ile Leu Ala Ile
65                  70                  75                  80
Glu Gln Tyr Glu Glu Val Val Asp Val Asn Cys Ser Ala Val Leu Arg
                85                  90                  95
Phe Phe Leu Cys Ala Met Tyr Ala Pro Ile Cys Thr Leu Glu Phe Leu
            100                 105                 110
His Asp Pro Ile Lys Pro Cys Lys Ser Val Cys Gln Arg Ala Arg Asp
        115                 120                 125
Asp Cys Glu Pro Leu Met Lys Met Tyr Asn His Ser Trp Pro Glu Ser
    130                 135                 140
Leu Ala Cys Asp Glu Leu Pro Val Tyr Asp Arg Gly Val Cys Ile Ser
145                 150                 155                 160
Pro Glu Ala Ile Val Thr Asp Leu Pro Glu Asp Val Lys Trp Ile Asp
                165                 170                 175
Ile Thr Pro Asp Met Met Val Gln Glu Arg Pro Leu Asp Val Asp Cys
            180                 185                 190
Lys Arg Leu Ser Pro Asp Arg Cys Lys Cys Lys Lys Val Lys Pro Thr
        195                 200                 205

Leu Ala Thr Tyr Leu Ser Lys Asn Tyr Ser Tyr Val Ile His Ala Lys
    210                 215                 220
Ile Lys Ala Val Gln Arg Ser Gly Cys Asn Glu Val Thr Thr Val Val
225                 230                 235                 240
Asp Val Lys Glu Ile Phe Lys Ser Ser Ser Pro Ile Pro Arg Thr Gln
                245                 250                 255
Val Pro Leu Ile Thr Asn Ser Ser Cys Gln Cys Pro His Ile Leu Pro
            260                 265                 270
His Gln Asp Val Leu Ile Met Cys Tyr Glu Trp Arg Ser Arg Met Met
        275                 280                 285
Leu Leu Glu Asn Cys Leu Val Glu Lys Trp Arg Asp Gln Leu Ser Lys
    290                 295                 300
Arg Ser Ile Gln Trp Glu Glu Arg Leu Gln Glu Gln Arg Arg Thr Val
305                 310                 315                 320
Gln Asp Lys Lys Lys Thr Ala Gly Arg Thr Ser Arg Ser Asn Pro Pro
                325                 330                 335
Lys Pro Lys Gly Lys Thr Pro Ala Pro Lys Pro Ala Ser Pro Lys Lys
            340                 345                 350
Asn Ile Lys Thr Arg Ser Ala Gln Lys Arg Thr Asn Pro Lys Arg Val
        355                 360                 365

(2) INFORMATION FOR SEQ ID NO:3:

(i) SEQUENCE CHARACTERISTICS:
(A) LENGTH: 1969 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:3:

CGCGGAGTCC GGGACTGGAG CTGCCCGGGC GGGTTCGCGC CCCGAAGGCT GAGAGCTGGC      60
GCTGCTCGTG CCCTGTGTGC CAGACGGCGG AGCTCCGCGG CCGGACCCCG CGGCCCCGCT     120
TTGCTGCCGA CTGGAGTTTG GGGGAAGAAA CTCTCCTGCG CCCCAGAGGA TTTCTTCCTC     180
GGCGAAGGGA CAGCGAAAGA TGAGGGTGGC AGGAAGAGAA GGGCGCTTTC TGTCTGCCGG     240
GGTCGCAGCG CGAGANGGCA GTGCCATGTT CCTCTCCATC CTAGTGGCGC TGTGCCTGTG     300
GCTGTCACCT GGGGCTGGGC GTGTCGCGGC GCCCCTGACG AGGTCGGTGC GCATCCCTAT     360
GTGCCGGCAC ATGCCCTGGA ACATCACGCG GATGCCCAAC CACCTGCACC ACAGCACGCA     420
GGAGAACGCC ATCCTGGCCA TCGAGCAGTA CGAGGAGCTG GTGGACGTGA ACTGCAGCGC     480
CGTGCTGCGC TTCTTCCTCT GTGCCATGTA CGCGCCCATT TGCACCCTGG AGTTCCTGCA     540
CGACCCTATC AAGCCGTGCA AGTCGGTGTG CCAACGCGCG CGCGACGACT GCGAGCCCCT     600
CATGAAGATG TACAACCACA GCTGGCCCGA AAGCCTGGCC TGCGACGAGC TGCCTGTCTA     660

TGACCGTGGC GTGTGCATCT CGCCTGAAGC CATCGTCACG GACCTCCCGG AGGATGTTAA 720
GTGGATAGAC ATCACACCAG ACATGATGGT ACAGGAAAGG CCTCTTGATG TTGACTGTAA 780
ACGCCTAAGC CCCGATCGGT GCAAGTGTAA AAAGGTGAAG CCAACTTTGG CAACATATCT 840
CAGCAAAAAC TACAGCTATG TTATTCATGC CAAAATAAAA GCTGTGCAGA GGAGTGGCTG 900
CAATGAGGTC ACAACGGTGG TGGATGTAAA AGAGATCTTC AAGTCCTCAT CACCCATCCC 960
TCGAACTCAA GTCCCGCTCA TTACAAATTC TTCTTGCCAG TGTCCACACA TCCTGCCCCA 1020
TCAAGATGTT CTCATCATGT GTTACGAGTG GCGCTCAAGG ATGATGCTTC TTGAAAATTG 1080
CTTAGTTGAA AAATGGAGAG ATCAGCTTAG TAAAAGATCC ATACAGTGGG AAGAGAGGCT 1140
GCAGGAACAG CGGAGAACAG TTCAGGACAA GAAGAAAACA GCCGGGCGCA CCAGTCGTAG 1200
TAATCCCCCC AAACCAAAGG GAAAGACTCC TGCTCCCAAA CCAGCCAGTC CCAAGAAGAA 1260
CATTAAAACT AGGGGTCGAC CCACGCGTCC GAAGAGAACA AACCCGAAAA GAGTGTGAGC 1320
TAACTAGTTT CCAAAGCGGA GACTTCCGAC TTCCTTACAG GATGAGGCTG GGCATTGCCT 1380
GGGACAGCCT ATGTAAGGCC ATGTGCCCCT TGCCCTAACA ACTCACTGCA GTGCTCTTCA 1440
TAGACACATC TTGCAGCATT TTTCTTAAGG CTATGCTTCA GTTTTTCTTT GTAAGCCATC 1500
ACAAGCCATA GTGGTAGGTT TGCCCTTTGG TACAGAAGGT GAGTTAAAGC TGGTGGAAAA 1560
GGCTTATTGC ATTGCATTCA GAGTAACCTG TGTGCATACT CTAGAAGAGT AGGGAAAATA 1620
ATGCTTGTTA CAATTCGACC TAATATGTGC ATTGTAAAAT AAATGCCATA TTTCAAACAA 1680
AACACGTAAT TTTTTTACAG TATGTTTATT ACCTTTTGAT ATCTGTTGTT GCAATGTTAG 1740
TGATGTTTAA AATGTGATCG AAAATATAAT GCTTCTAAGA AGGAACAGTA GTGGGAATGA 1800
ATGTCTAAAA GATCTTTATG TGTTTATGGT CTGCCAGAAG GATTTTTGTG ATGAAAGGGG 1860
ATTTTTTGAA AAATCTAGGG GAAGTAGCCA TATGGGAAAA TTATNATGTG TCTTTTTTAC 1920
ATGGACTTCC AGCTCCGTTT TTTGGCTNGG AAACTCTNAA AACCAAANT 1969

(2) INFORMATION FOR SEQ ID NO:4:

(i) SEQUENCE CHARACTERISTICS:
(A) LENGTH: 372 amino acids
(B) TYPE: amino acid
(C) STRANDEDNESS: single
(D) TOPOLOGY: linear
(ii) MOLECULE TYPE: protein

(xi) SEQUENCE DESCRIPTION: SEQ ID NO:4:

Met Arg Val Ala Gly Arg Glu Gly Arg Phe Leu Ser Ala Gly Val Ala
1               5                   10                  15
Ala Arg Xaa Gly Ser Ala Met Phe Leu Ser Ile Leu Val Ala Leu Cys
            20                  25                  30
Leu Trp Leu Ser Pro Gly Ala Gly Arg Val Ala Ala Pro Leu Thr Arg
        35                  40                  45
Ser Val Arg Ile Pro Met Cys Arg His Met Pro Trp Asn Ile Thr Arg
    50                  55                  60
Met Pro Asn His Leu His His Ser Thr Gln Glu Asn Ala Ile Leu Ala

65                  70                  75                  80
Ile Glu Gln Tyr Glu Glu Leu Val Asp Val Asn Cys Ser Ala Val Leu
                85                  90                  95
Arg Phe Phe Leu Cys Ala Met Tyr Ala Pro Ile Cys Thr Leu Glu Phe
            100                 105                 110
Leu His Asp Pro Ile Lys Pro Cys Lys Ser Val Cys Gln Arg Ala Arg
        115                 120                 125
Asp Asp Cys Glu Pro Leu Met Lys Met Tyr Asn His Ser Trp Pro Glu
    130                 135                 140
Ser Leu Ala Cys Asp Glu Leu Pro Val Tyr Asp Arg Gly Val Cys Ile
145                 150                 155                 160
Ser Pro Glu Ala Ile Val Thr Asp Leu Pro Glu Asp Val Lys Trp Ile
                165                 170                 175
Asp Ile Thr Pro Asp Met Met Val Gln Glu Arg Pro Leu Asp Val Asp
            180                 185                 190
Cys Lys Arg Leu Ser Pro Asp Arg Cys Lys Cys Lys Lys Val Lys Pro
        195                 200                 205
Thr Leu Ala Thr Tyr Leu Ser Lys Asn Tyr Ser Tyr Val Ile His Ala
    210                 215                 220
Lys Ile Lys Ala Val Gln Arg Ser Gly Cys Asn Glu Val Thr Thr Val
225                 230                 235                 240
Val Asp Val Lys Glu Ile Phe Lys Ser Ser Ser Pro Ile Pro Arg Thr
                245                 250                 255
Gln Val Pro Leu Ile Thr Asn Ser Ser Cys Gln Cys Pro His Ile Leu
            260                 265                 270
Pro His Gln Asp Val Leu Ile Met Cys Tyr Glu Trp Arg Ser Arg Met
        275                 280                 285
Met Leu Leu Glu Asn Cys Leu Val Glu Lys Trp Arg Asp Gln Leu Ser
    290                 295                 300
Lys Arg Ser Ile Gln Trp Glu Glu Arg Leu Gln Glu Gln Arg Arg Thr
305                 310                 315                 320
Val Gln Asp Lys Lys Lys Thr Ala Gly Arg Thr Ser Arg Ser Asn Pro
                325                 330                 335
Pro Lys Pro Lys Gly Lys Thr Pro Ala Pro Lys Pro Ala Ser Pro Lys
            340                 345                 350
Lys Asn Ile Lys Thr Arg Gly Arg Pro Thr Arg Pro Lys Arg Thr Asn
        355                 360                 365
Pro Lys Arg Val
    370